(19) Europäisches Patentamt
European Patent Office
Office européen des brevets

(11) **EP 2 294 972 B1**

(12) **EUROPEAN PATENT SPECIFICATION**

(45) Date of publication and mention
of the grant of the patent:
**22.08.2012  Bulletin 2012/34**

(51) Int Cl.:
***A61B 5/00*** *(2006.01)*    ***A61B 5/0205*** *(2006.01)*
***A61B 5/0452*** *(2006.01)*

(21) Application number: **10251578.0**

(22) Date of filing: **10.09.2010**

(54) **System comprising an implantable medical device for detecting stroke based on physiological and electrocardiac indices**

System mit einer implantierbaren medizinischen Vorrichtung zur Erkennung von Schlaganfällen basierend auf physiologischen und elektrokardialen Indices

Système comprenant un dispositif médical implantable de détection d'accident vasculaire cérébral par les indices physiologiques et électrocardiaques

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **11.09.2009  US 558385**

(43) Date of publication of application:
**16.03.2011  Bulletin 2011/11**

(73) Proprietor: **PACESETTER, INC.**
**Sylmar, CA 91392-9221 (US)**

(72) Inventors:
  • **Bharmi, Rupinder**
    **Canyon Country, CA 91387 (US)**
  • **Farazi, Taraneh Ghaffari**
    **Santa Clara, CA 95120 (US)**

(74) Representative: **Phillips, Emily Elizabeth et al**
    **Kilburn & Strode LLP**
    **20 Red Lion Street**
    **London WC1R 4PJ (GB)**

(56) References cited:
**EP-A1- 2 215 965      DE-A1-102007 045 738**
**US-A1- 2007 273 504**

• **GUJJAR ARUNODAYA R ET AL: "Heart rate variability and outcome in acute severe stroke: Role of power spectral analysis", NEUROCRITICAL CARE, vol. 1, no. 3, 2004, pages 347-353, XP002615195, ISSN: 1541-6933**
• **GIUSEPPE MICIELI ET AL: "The autonomic nervous system and ischemic stroke: a reciprocal interdependence", CLINICAL AUTONOMIC RESEARCH, vol. 18, no. 6, 11 October 2008 (2008-10-11), pages 308-317, XP019637460, ISSN: 1619-1560, DOI: 10.1007/S10286-008-0495-7**

**Description**

FIELD OF THE INVENTION

The invention generally relates to implantable medical devices, such as pacemakers and implantable cardioverter/defibrillators (ICDs) and, in particular, to techniques for detecting and responding to stroke within patients in which such devices are implanted.

BACKGROUND OF THE INVENTION

A stroke is a sudden loss of brain function caused by a blockage of a blood vessel to the brain (ischemic stroke) or a rupture of a blood vessel to the brain (hemorrhagic stroke). Each year, more than 700,000 people in the U.S. suffer a new or recurrent stroke and the consequences can be devastating. Some victims are physically and mentally devastated and unable to move, speak, or eat normally. About 15% of those who have a stroke die in the hospital. The proportion is even higher among the elderly.

Although the consequences of a stroke do not appear to be reversible, early intervention (particularly within the first one to three hours) can significantly reduce morbidity and mortality. Moreover, the degree of long-term impairment as a result of stroke can typically be reduced through prompt intervention. Accordingly, it would be highly desirable to provide techniques for detecting the onset of a stroke within a patient and for promptly alerting family members, caregivers or emergency personnel, and it is to that end that the invention is generally directed. It would also be desirable to automatically deliver therapy in response to the stroke to mitigate harm.

As many patients at risk of stroke are candidates for implantable medical devices such as pacemakers and ICDs, it would be particularly desirable to equip such devices with the capability to detect and respond to stroke. This is especially important for patients known to suffer from atrial fibrillation (AF) as these patients are at particular risk of having a stroke.

DE-A-10 2007 045 738 discloses a system for early detection of stroke, wherein a chest belt is provided for sensing ECG signals. Using RR interval analysis, at least one parameter is extracted for detecting AF.

EP-A-2 215 965, which is a document falling under Article 54(3) EPC, discloses systems and methods for use with an implantable medical device for detecting stroke based on electrocardiac signals. The implantable medical device detects a preliminary indication of a possible stroke based on the electrocardiac signals and transmits a signal indicative of possible stroke to an external system. The external system then generates a stroke questionnaire for use in confirming the stroke. In other implementations, the implanted stroke monitor detects the stroke solely based on the ECG signals, rather than relying on a questionnaire to confirm the stroke. Exemplary changes indicative of stroke include the onset of prominent U-waves, the onset of notched T-waves, changes in ST segment duration, and changes in QT duration and/or trends in any of these signals.

The invention is set out in the claims.

For detecting a stroke event, electrocardiac and physiological signals are sensed within the patient. A plurality of indices is derived from the electrocardiac signals and the physiological signals based on parameters affected by stroke. A stroke event is then detected within the patient based on an examination of the plurality of indices. Warnings are generated, neurostimulation is delivered, pacing therapy is adjusted, medications are delivered and/or other device functions are controlled in response to the indication of the stroke.

In one particular example, the electrocardiac signals sensed by the device include intracardiac electrograms (IEGMs) obtained within various chambers of the heart. The physiological signals include arterial and systolic blood pressure signals. The following indices are derived from these signals: a heart rate variability index (HRV_Index); a heart rate turbulence index (HRT_Index); a baroreflex index (BRS_Index); a QT index (QT_Index); a respiration index (RESP_Index); and a circadian variability index (CV_Index). Also, time delta indices are generated for each index representative of a time rate of change of the index.

The device then generates a composite stroke index from the plurality of separate indices using, e.g., the following equation:

$$\text{Composite Stroke Index } =$$

$$\text{HRV\_Index / HRVtime\_Delta\_Index} \quad +$$

$$\text{HRT\_Index / HRTtime\_Delta\_Index} \quad +$$

$$\text{BRS\_Index / BRStime\_Delta\_Index} \quad +$$

$$\text{QT\_Index / QTtime\_Delta\_Index} \quad +$$

$$\text{RESP\_Index / RESPtime\_Delta\_Index} \quad +$$

$$\text{CV\_Index / CVtime\_Delta\_Index,}$$

The device compares the composite index against a threshold indicative of a stroke event. If stroke is indicated, the device can respond by generating both internal warning signals for alerting the patient and external warning signals (via bedside monitors or the like) for notifying family members, caregivers or emergency personal. In particular, the device can notify urgent care centers for immediate response.

If so equipped, the device can deliver neurostimulation to the patient in an effort to improve heart rate and blood pressure in response to the stroke. Such stimulation can include, e.g., spinal cord stimulation, baro-receptor stimulation and sympathetic nerve stimulation. Pacing can also be controlled in response to stroke. For example, rate smoothing or paired pacing can be activated. Any changes in pacing can be synchronized with real-time changes in blood pressure detected within the patient. Also, if equipped with an implantable drug dispensing system such as a drug infusion pump, medications can be delivered directly to the patient in response to the stroke.

In addition to controlling pacing and/or neurostimulation therapy in response to the onset of the acute stroke, such therapy can also be controlled to work in conjunction with physician-directed therapy for the patient.

The implantable device can also be used to track improvement in patient condition after the patient has had the stroke and is then undergoing treatment.

System examples of the invention are described herein below.

The above and further features, advantages and benefits of the invention will be apparent upon consideration of the descriptions herein taken in conjunction with the accompanying drawings, in which:

**FIG. 1** illustrates pertinent components of an implantable medical system capable of detecting the onset of a stroke based on electrocardiac and physiological signals sensed within the patient;

**FIG. 2** provides an overview of the method for detecting stroke performed by the system of **FIG. 1,** which exploits a set of indices derived from various electrocardiac and physiological signals sensed by the system of **FIG. 1;**

**FIG. 3** illustrates an exemplary embodiment of the general technique of **FIG. 2,** wherein a composite stroke index is derived from a combination of individual indices;

**FIG. 4** summarizes the derivation of the heart rate variability (HRV_Index) for use with the technique of **FIG. 3;**

**FIG. 5** is a graph illustrating the power spectra of heart rate variability for a healthy patient, portions of which are exploited in the derivation of the HRV_Index of **FIG. 4;**

**FIG. 6** is a graph illustrating the power spectra of heart rate variability for a patient after a stroke, illustrating the effect of the stroke on heart rate variability, which is quantified by the HRV_Index of **FIG. 4;**

**FIG. 7** summarizes the derivation of the heart rate turbulence index (HRT_Index) for use with the technique of **FIG. 3;**

**FIG. 8** summarizes the derivation of the baroreflex index (BRS_Index) for use with the technique of **FIG. 3;**

**FIG. 9** summarizes the derivation of the QT index (QT_Index) for use with the technique of **FIG. 3;**

**FIG. 10** summarizes the derivation of the respiration index (RESP_Index) for use with the technique of **FIG. 3;**

**FIG. 11** summarizes the derivation of the circadian variability index (CV_Index) for use with the technique of **FIG. 3;**

**FIG. 12** is a graph illustrating circadian variations in high frequency components of heart rate variability for a healthy patient and a patient after a stroke, illustrating the effect of the stroke on the circadian variability, which is quantified by the CV_Index of **FIG. 11;**

**FIG. 13** summarizes the derivation of the composite stroke index for use with the technique of **FIG. 3;**

**FIG. 14** is a simplified, partly cutaway view, illustrating the pacer/ICD of **FIG. 1** along with at set of leads implanted into the heart of the patient; and

**FIG. 15** is a functional block diagram of the pacer/ICD of **FIG. 14,** illustrating basic circuit elements that provide cardioversion, defibrillation and/or pacing stimulation in the heart and particularly illustrating components for detecting and responding to stroke using the techniques of **FIGS. 2 - 13.**

DETAILED DESCRIPTION OF THE PREFERRED EMBODIMENTS

The following description includes the best mode presently contemplated for practicing the invention. This description is not to be taken in a limiting sense but is made merely to describe general principles of the invention. The scope of the invention is defined by the appended claims. In the description of the invention that follows, like numerals or reference designators are used to refer to like parts or elements throughout.

Overview of Implantable Medical System

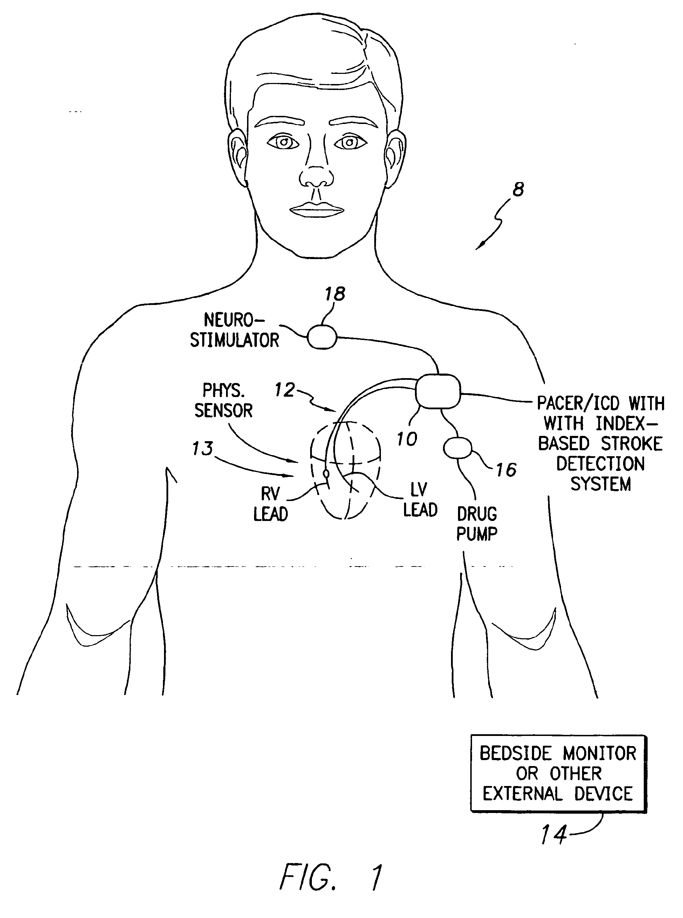

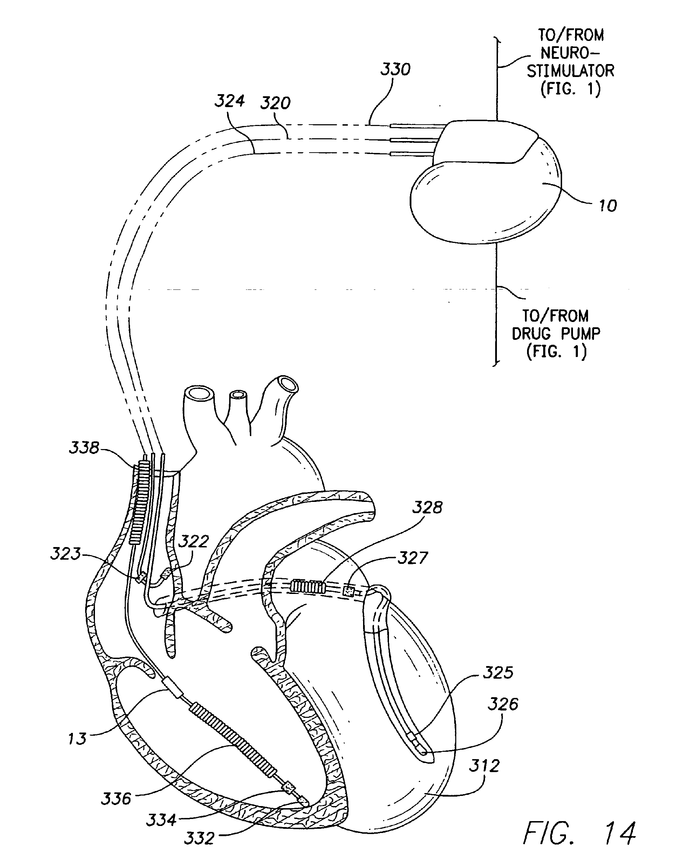

**FIG. 1** illustrates an implantable medical system 8 capable of detecting a cerebral stroke within a patient based on indices derived from electrocardiac and physiological signals. To this end, medical system 8 includes a pacer/ICD 10 or other cardiac rhythm management (CRM) device capable of detecting electrocardiac signals via cardiac sensing/pacing leads 12 implanted within the heart of the patient. The leads may be equipped with blood pressure sensors, photo-plethysmography (PPG) sensors, or other physiological sensors. One such physiological sensor 13 is shown implanted in the RV. This is merely exemplary. Other sensors may be implanted at other locations, in or around the heart or elsewhere within the patient. Note that in **FIG. 1** only two exemplary leads are shown -- an RV lead and an LV lead, in stylized form. A more complete set of leads is illustrated in **FIG. 14.**

Warning signals may be generated to warn of stroke using an internal warning device within the pacer/ICD, a bedside monitor 14, or a handheld personal advisory module (PAM), not separately shown. The internal warning device (not shown in **FIG. 1**) may be a vibrating device or a "tickle" voltage device that, in either case, provides perceptible stimulation to the patient to alert the patient. The bedside monitor or PAM then alerts family members or caregivers of the stroke and may also forward warning signals or other suitable information via a centralized processing system 14 to the patient's primary care physician or, in some implementations, directly to emergency personnel. Such an alarm would enable the patient/family to recognize the stroke early, so that the patient can be taken to the emergency care immediately. The centralized system may include such systems as the HouseCall™ system or the Merlin@home/Merlin.Net/Merlin Mobile systems of St. Jude Medical.

In some examples, the implantable system may be equipped with a drug pump 16 capable of the delivering medications to patient tissues in response to the stroke. Implantable drug pumps for use in dispensing medications are discussed in U. S. Patent 5,328,460 to Lord, et al.*,* entitled "Implantable Medication Infusion Pump Including Self-Contained Acoustic Fault Detection Apparatus." This patent also discusses implantable "tickle" warning devices that may be used to deliver warning signals to the patient.

Diagnostic information pertaining to the stroke may be stored within the pacer/ICD for transmission to the PAM, bedside monitor or to an external programmer (not shown in **FIG. 1**) for review by a clinician. The clinician then prescribes any appropriate therapies. The clinician may also adjust the operation of the pacer/ICD to activate, deactivate or otherwise control any therapies that are automatically applied.

The pacer/ICD may also be programmed to activate, adjust or control any pacing therapies that might be appropriate in response to stroke, such as activating rate smoothing or paired pacing. Still further, if equipped with suitable neurostimulators (such as a spinal cord neurostimulator 18) neurostimulation can be selectively applied to nerves of the patient in response to stroke. Additionally, the pacer/ICD performs a wide variety of pacing and/or defibrillation functions such as delivering pacing is response to arrhythmias or generating and delivering shocks in response to ventricular fibrillation.

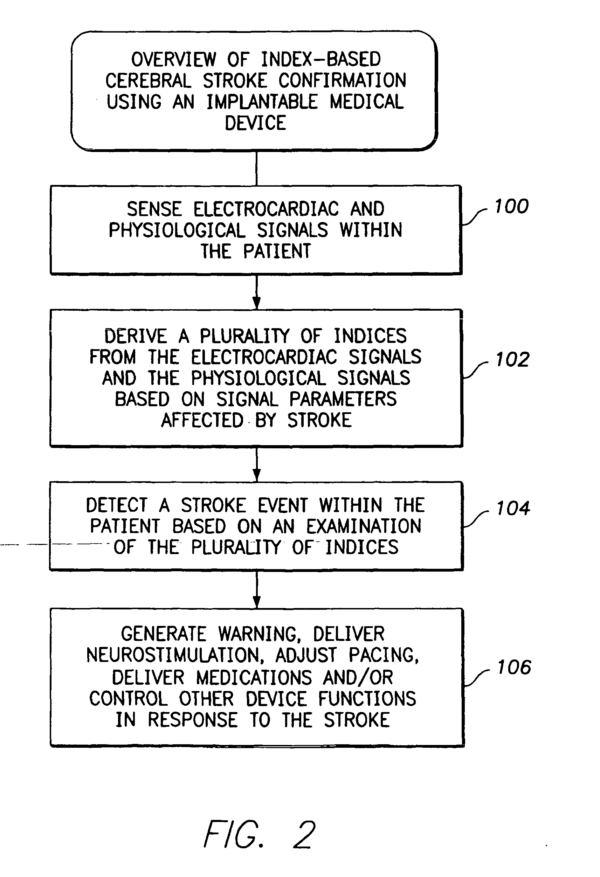

**FIG. 2** broadly summarizes a general technique for detecting stroke that may be performed by the implantable system of **FIG. 1** or other suitably equipped systems. Beginning at step 100, the pacer/ICD senses electrocardiac and physiological signals within the patient and, at step 102, derives a plurality of indices from the electrocardiac and physiological signals based on parameters affected by stroke. As will be explained in greater detail, such indices include a heart rate variability index, a heart rate turbulence index, a baroreflex index, etc. At step 104, the pacer/ICD then detects a stroke event within the patient based on an examination of the plurality of indices. In one example, the indices are combined into a composite stroke index for comparison against a threshold indicative of a stroke event. At step 106, the device generates warnings, delivers neurostimulation, adjusts pacing, delivers medications and/or controls other device functions in response to the stroke.

Hence, **FIGS. 1 and 2** provide an overview of an implantable system and method capable of detecting stroke within a patient and further capable of controlling pacing, delivering medications, control neurostimulation, delivering warnings, etc., in response to the stroke. Embodiments may be implemented that do not necessarily perform all of these functions. For example, embodiments may be implemented that provide only for generating warnings in response to stroke without necessarily delivering medications or controlling therapy. In addition, systems provided in accordance with the invention need not include all the components shown in **FIG. 1,** such as the bedside monitor, the implantable drug pump or the neuro-stimulator. No attempt is made herein to describe all possible combinations of components that may be provided in accordance with the general principles of the invention. Also, note that, the particular shape, size and locations of the implanted components shown in **FIG. 1** are merely illustrative and may not necessarily correspond to actual implant locations. In particular, preferred implant locations for the leads are more precisely illustrated in **FIG.14.**

Exemplary Index-based Stoke Detection Technique

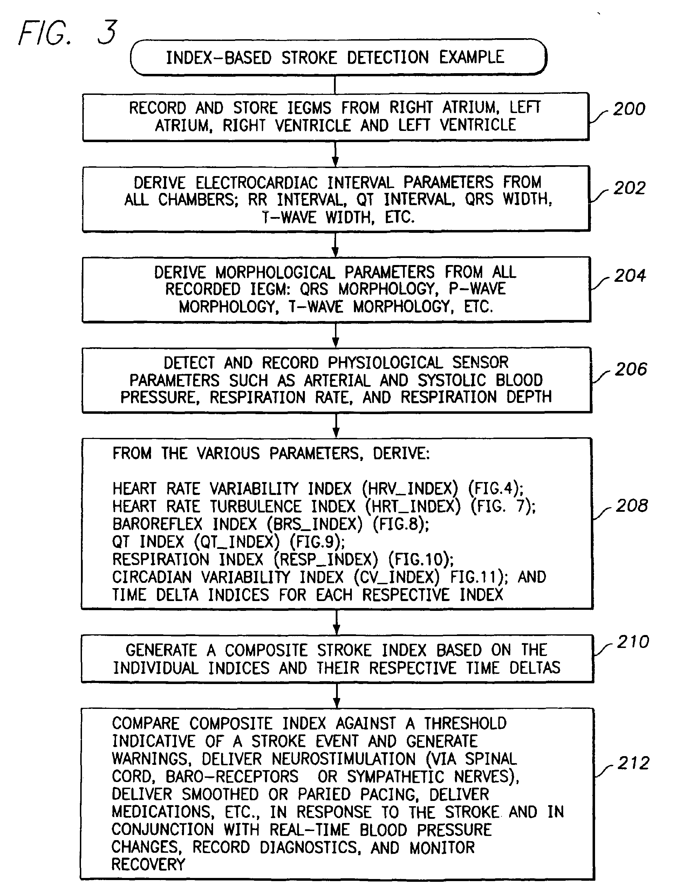

**FIG. 3** illustrates an exemplary index-based stroke detection technique wherein stroke indices and time delta indices are exploited. Beginning at step 200, the pacer/ICD senses, records and stores IEGMs from the right atrium (RA), right ventricle (RV), left ventricle (LV) and the left atrium (LA) of the patient. With a set of bipolar or multipolar leads, numerous sensing vectors can be exploited. For the purposes of this example, it is assumed that at least one IEGM is sensed and stored for each of the RA, LA, RV, and LV.

At step 202, the pacer/ICD derives electrocardiac interval parameters from all four chambers, including RR interval, QT interval, QRS width and T-wave width. Other interval parameters that might also be derived include PP intervals, PR intervals, ST intervals and P-wave width.

At step 204, the pacer/ICD derives morphological parameters from all IEGMs, including parameters representative of QRS morphology, P-wave morphology, and T-wave morphology. Specific morphological parameters can include the waveform amplitude, slope, integral, etc.

At step 206, the pacer/ICD detects and records physiological sensor parameters such as arterial and systolic blood pressure, respiration rate and respiration depth. Systolic blood pressure can be detected using, e.g., a PPG sensor. PPG sensors are discussed in U.S. Patent Application 11/927,026, filed Oct., 29, 2007, entitled "Systems and Methods for Exploiting Venous Blood Oxygen Saturation in Combination with Hematocrit or other Sensor Parameters for use with an Implantable Medical Device." Arterial blood pressure detection techniques are discussed in U.S. Patent Application Serial No. 11/378,604, filed March 16, 2006, of Kroll et al., entitled, "System and Method for Detecting Arterial Blood Pressure based on Aortic Electrical Resistance using an Implantable Medical Device." Particularly effective techniques for integrating one or more physiological sensors into pacing/sensing leads are set forth in U.S. Patent Application Serial No. 11/623,663, filed January 16, 2007, of Zou et al., entitled "Sensor/Lead Systems for use with Implantable Medical Devices."

Respiration parameters can be detected using any of a variety of techniques. For example, IEGMs can be exploited. See, U.S. Patent 7,361,146, to Bharmi, et al., entitled "System and Method for Detecting Abnormal Respiration via Respiratory Parameters derived from Intracardiac Electrogram Signals." Also, impedance can be used to track respiration. See, e.g., U.S. Patent No. 6,449,509 to Park, et al., entitled "Implantable Stimulation Device having Synchronous Sampling for a Respiration Sensor." Respiration parameters can also be obtained from heart sounds. See, e.g., U.S. Patent No. 6,064,910 to Andersson, et al., entitled "Respirator Rate/Respiration Depth Detector and Device for Monitoring Respiratory Activity Employing Same."

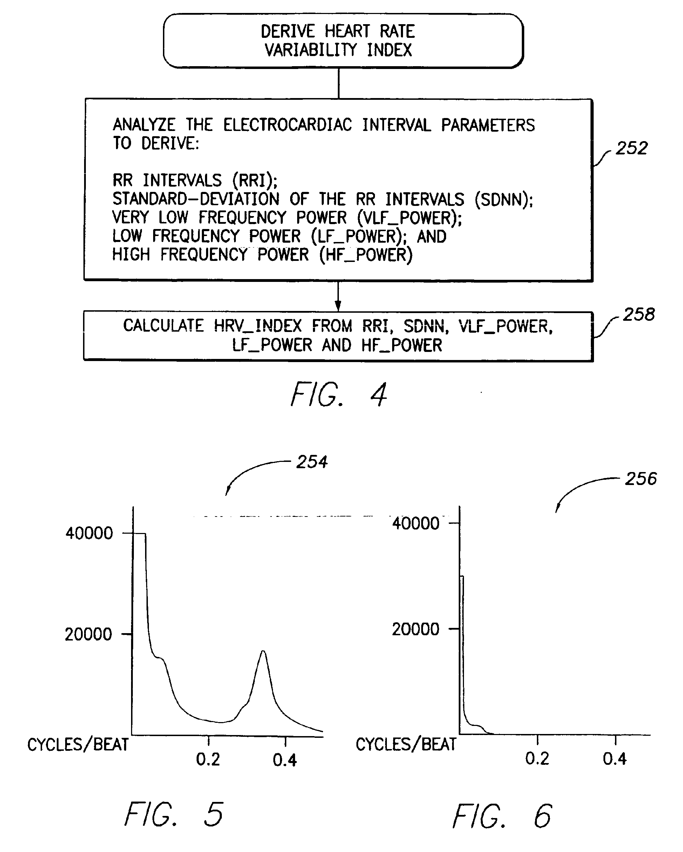

At step 208, the pacer/ICD then derives from the various parameters obtained at steps 202 - 206: a Heart Rate Variability Index (HRV_Index); a Heart Rate Turbulence Index (HRT_Index); a Baroreflex Index (BRS_Index); a QT Index (QT_Index); a Respiration Index (RESP_Index); a Circadian Variability Index (CV_Index); and a Time Delta Index for each individual Index. Techniques for deriving these particular indices are discussed below with reference to **FIGS. 4 - 12.** It should be understood that, in some examples, not all of these indices are calculated. In other examples, additional or alternative indices can be used.

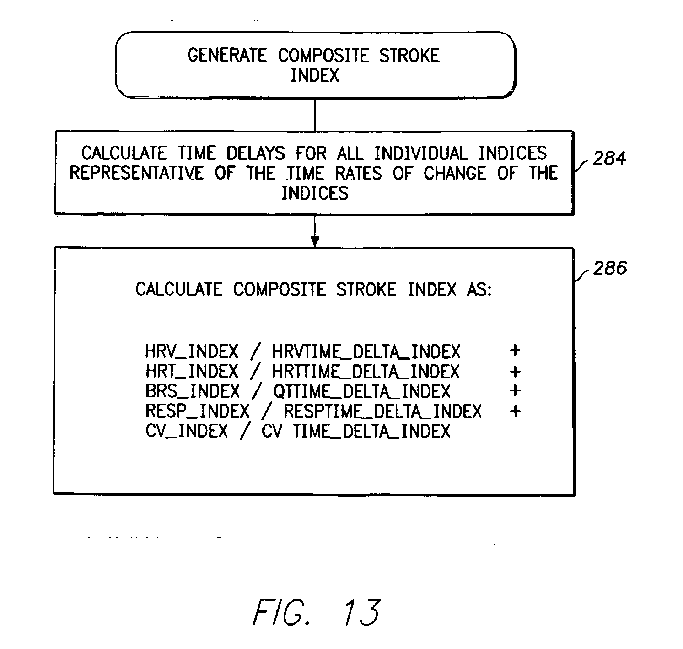

At step 210, the pacer/ICD then generates a composite stroke index based on the various individual indices and their respective time deltas. This is described in greater detail below with reference to **FIG. 13.** At step 212, the pacer/ICD compares the composite index against a threshold indicative of stroke event. If the index exceeds the threshold, a cerebral stroke is thereby detected. If stroke is detected, the pacer/ICD generates warnings, delivers neurostimulation, delivers smoothed or paired pacing, delivers medications, records diagnostics, etc.

It is particularly important to restore blood pressure and heart rate following a stroke. A combination of neurostimulator and cardiac pacing can prevent help hemodynamic compromise of the patient to reduce the impact to the patient by closely tying the pacing scheme to real-time pressure. Hence, in some examples, therapy is delivered in conjunction with (or synchronized with) real-time blood pressure changes. For example, if the blood pressure is particularly low, certain types of therapy might be delivered that would not otherwise be delivered if the blood pressure were normal. In this regard, a major concern during stroke is maintenance of blood pressure. It is recommended that the blood pressure should not be low (e.g. less than 120 mmHg), but not too high either (e.g. not greater than 180mmHg.) Multiple thresholds of differing levels may be used to evaluate the severity of the stroke and, in some examples, the response by the pacer/ICD to the stroke depends on the level of severity.

As noted above, warnings may be relayed through external systems or networks to family members, caregivers, emergency personnel, etc.

Neurostimulation may be delivered via the spinal cord, baroreceptors or sympathetic nerves, depending upon the capabilities of the device. Spinal cord stimulation via an implantable lead is discussed, e.g., in U.S. Patent 7,099,718, to Thacker, et al. Baro-receptor stimulation to control blood pressure is discussed in U.S. Patent No. 6,050,952 to Hakki, et al. Techniques for stimulating sympathetic nerves are discussed in U.S. Patent No. 6,937,896, to Kroll, entitled, "Sympathetic Nerve Stimulator and/or Pacemaker."

Rate smoothed pacing is discussed in U.S. Patent No. 6,636,766, Bornzin, et al., "System and Method for Preventing Sudden Heart Rate Drop using Implantable Cardiac Pacing Device." Paired pacing is discussed in U.S. Patent Application No. US 2010 094 371, of Bornzin et al., filed Oct. 30, 2007, entitled "Systems and Methods for Paired/ Coupled Pacing and Dynamic Overdrive/Underdrive Pacing."

Insofar as delivery of medications are concerned, tPA tissue plasminogen activator or like compounds can be used to help restore blood flow to the brain immediately following are stroke. (Note that tPA tissue plasminogen activator is a thrombolytic agent, i.e. a compound for breaking down clots). After the stroke is completed, anticoagulants can be given to prevent subsequent stokes, particularly in patients known to have atrial fibrillation or a heart-valve disorder. Suitable versions of these or other compounds may be identified for dispensing via an implantable drug dispensing unit, drug infusion unit and/or drug pump under the control of the pacer/ICD. See, e.g., U.S. Patent No. 7,235,530, to Blair, et al., entitled "Kallikrein Inhibitors and Anti-Thrombolytic Agents and Uses Thereof," which discusses compounds suitable for delivery via a medication infusion pump.

If the device is equipped with an alternative or secondary stroke detection system, it might be desirable in some implementations to confirm the detection of stroke using the alternative system before therapy is delivered. See, for example, the aforementioned patent application of Park, which discusses the use of U-waves, notched T-waves, and changes in ST segment duration or QT duration to provide a preliminary indication of stroke. (As noted above, the system of Park uses an external questionnaire-based confirmation system wherein family members or other caregivers input answers to a questionnaire into an external system, which then confirms the stroke. As the present invention is directed to providing prompt detection of stroke by the pacer/ICD itself, a questionnaire is not used.)

Note also that, in addition to controlling pacing and/or neurostimulation therapy in response to the onset of the acute stroke, therapy can also be controlled to work in conjunction with physician-directed therapy for the patient. The pacer/ICD can also be equipped to detect and record diagnostic information and to monitor for improvement in patient condition after the patient has had stroke and is undergoing treatment. For example, changes in the composite stroke index may be periodically recorded as an indication of patient recovery.

Turning now to **FIGS. 4 - 12,** exemplary techniques for deriving the individual indices will now be described.

**FIG. 4** summarizes derivation of the heart rate variability index. A cerebral infarction located particularly in the hemispheric level or in the medullary oblongata causes cardiovascular autonomic dysregulation manifesting itself as impaired heart rate variability. Heart rate variability parameters correlate with functional independence measures in ischemic stroke patients. The values of HRV stay significantly lower after the acute phase of the disease in patients who have suffered ischemic stroke compared to healthy persons of the same age. Moreover, impaired heart rate variability correlates with the severity of neurological deficits and disability.

Accordingly, at step 250, the pacer/ICD analyzes the electrocardiac interval parameters detected at step 202 of **FIG. 3** to extract or derive: a set of recently detected RR intervals (RRI), such as the last 256 RR intervals or the last 4 minutes worth of RR interval data; the standard-deviation of the set of RR intervals (SDNN); a very low frequency power component of the set of RR intervals (VLF_POWER); a low frequency power component of the set of RR intervals (LF_POWER); and a high frequency power component of the set of RR intervals (HF_POWER). To this end, a Fast-Fourier Transform or other suitable technique may be employed to derive power spectra from the RR interval data, resulting in a power spectral curve of the type shown in **FIGS. 5 and 6.** The area under the spectral curve from 0.005 to 0.4 Hz represents the total power of heart rate variability, the area from 0.15 to 0.4 Hz represents HF power, the area from 0.04 to 0.15 Hz represents LF power, and the area from 0.005 to 0.04 Hz represents VLF power. See, e.g., Korpelainen et al., "Abnormal Heart Rate Variability as a Manifestation of Autonomic Dysfunction in Hemispheric Brain Infarction" Stroke. 1996;27:2059-2063.

More specifically, **FIG. 5** illustrates the power spectra 254 of RR intervals observed in a healthy 61-year-old male control subject. **FIG. 6** illustrates the power spectra 256 for a 61-year-old male patient four days after a right hemisphere brain infarction (i.e. stroke). As can be seen, all but the lowest frequency components of heart rate variability are suppressed in the stroke patient, indicative of very little variability in heart rate. As such, an index representative of the distribution of spectral components of heart rate variability can be indicative of stroke.

At step 258, the pacer/ICD calculates an HRV_Index from RRI, SDNN, VLF_POWER, LF_POWER AND HF_POWER by using an average or weighted average. In one example, the following equation is used to calculate the HRV_Index:

$$\text{HRV\_Index} = \quad a*RRI / \text{mean(Heart rate)} +$$
$$b*SDNN / \text{mean(Heart rate)} +$$
$$c*(VLF\_POWER + LF\_POWER +$$
$$HF\_POWER) / (3*\text{Total Power})$$

and where a = 10%, b = 20%, c =70%.

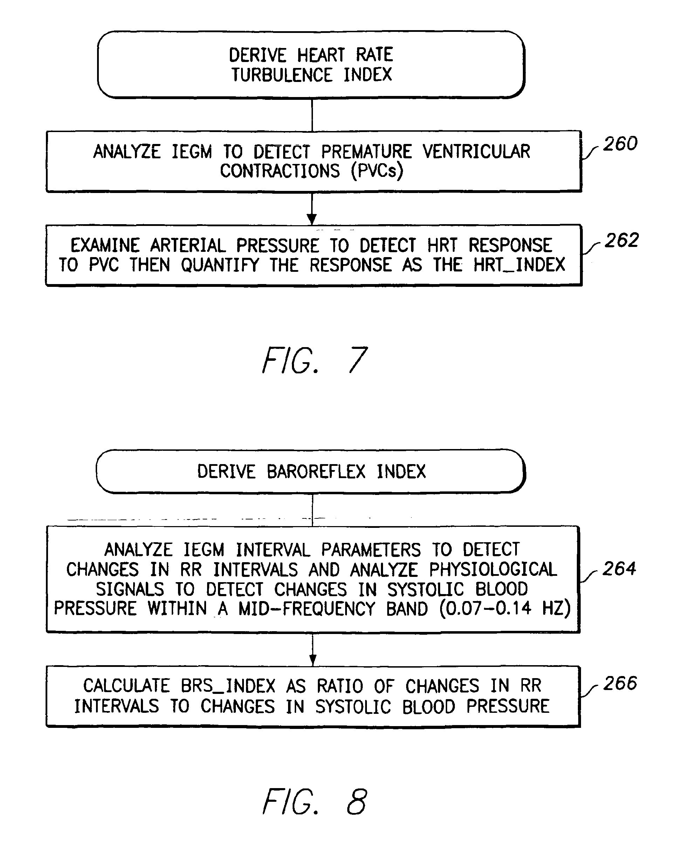

**FIG. 7** summarizes derivation of the heart rate turbulence index. HRT is an autonomous baro-reflex that is weakened/eliminated with stroke. HRT consists of a short initial acceleration followed by a deceleration of the heart rate following a premature ventricular contraction. The premature ventricular contraction causes a brief disturbance of the arterial blood pressure (low amplitude of the premature beat, high amplitude of the ensuing normal beat). When the autonomic control system of the patient is intact, this fleeting change is registered immediately with an instantaneous response in the form of HRT. If the autonomic control system is impaired, this reaction is either weakened or entirely missing.

Accordingly, at step 260, the pacer/ICD analyzes the IEGM signals detected at step 200 of **FIG. 3** to detect premature ventricular contractions (PVCs) using otherwise conventional techniques. Alternatively, PVCs can be generated by a premature RV paced beat. Then, at step 262, the pacer/ICD examines arterial pressure signals (detected at step 206 of FIG. 3) to detect HRT response to PVC, then quantifies the response as the HRT_Index. In one example, the following equation/procedure is used to calculate the HRT_Index:

$$\text{HRT\_Index} = (\text{Turbulence onset} + \text{Turbulence slope}$$
$$+ \text{Turbulence timing}) / \text{mean(heart rate)}.$$

That is, the quantification of HRT may be achieved via Turbulence onset and turbulence slope. Turbulence Onset (TO) is the percentage difference between the heart rate immediately following PVC and the heart rate immediately preceding PVC. It may be calculated using the equation:

$$TO = ((RR1 + RR2) - (RR\text{-}2 + RR\text{-}1)) / (RR\text{-}2 + RR\text{-}1) * 100$$

where RR-2 and RR-1 are the first two normal intervals preceding the PVC and RR1 and RR2 are the first two normal intervals following the PVC. Initially, TO is determined for each individual PVC, followed by the determination of the average value of all individual measurements. Positive values for Turbulence Onset indicate deceleration; negative values indicate acceleration of the sinus rhythm.

The Turbulence Slope (TS) corresponds to the steepest slope of the linear regression line for each sequence of five consecutive normal intervals in the local tachogram. The Turbulence Slope calculations are based on the averaged tachogram and expressed in ms per RR interval. Turbulence timing (TT) may be determined by the beat number of the 5 RR interval sequence used in the TS calculation at which the largest slope occurs.

See, for example, Watanabe, et al. "Effects of ventricular premature stimulus coupling interval on blood pressure and heart rate turbulence." Circulation. 2002 Jul 16;106(3):325-30.

FIG. 8 summarizes the derivation of the baroreflex (BRS) index. BRS decreases during exercise and mental activity and peaks during sleep. BRS can be measured by a ratio between changes in RR interval and changes in systolic blood pressure in a mid-frequency band (0.07-0.14Hz). Accordingly, at step 264, the pacer/ICD analyzes the IEGM interval parameters (detected at step 202 of **FIG. 3)** to detect changes in RR intervals over time and also analyzes physiological signals (detected at step 206 of **FIG. 3)** to detect changes in systolic blood pressure over time within a mid-frequency spectral band (0.07 - 0.14 HZ) derived from the systolic pressure signals. At step 266, the pacer/ICD then calculate the BRS_Index as a ratio of changes in RR intervals over time to changes in systolic blood pressure over time within the mid-frequency spectral band.

For example, changes in RR interval may be represented as $\Delta RR$. Changes in systolic pressure within the mid-frequency spectral band may be represented as $\Delta P_{sys}$.

$$\text{BRS\_Index} = (\Delta RR\ /\ \Delta P_{sys})\ /\ (\text{mean heart rate}\ /\ \text{mean systolic pressure})$$

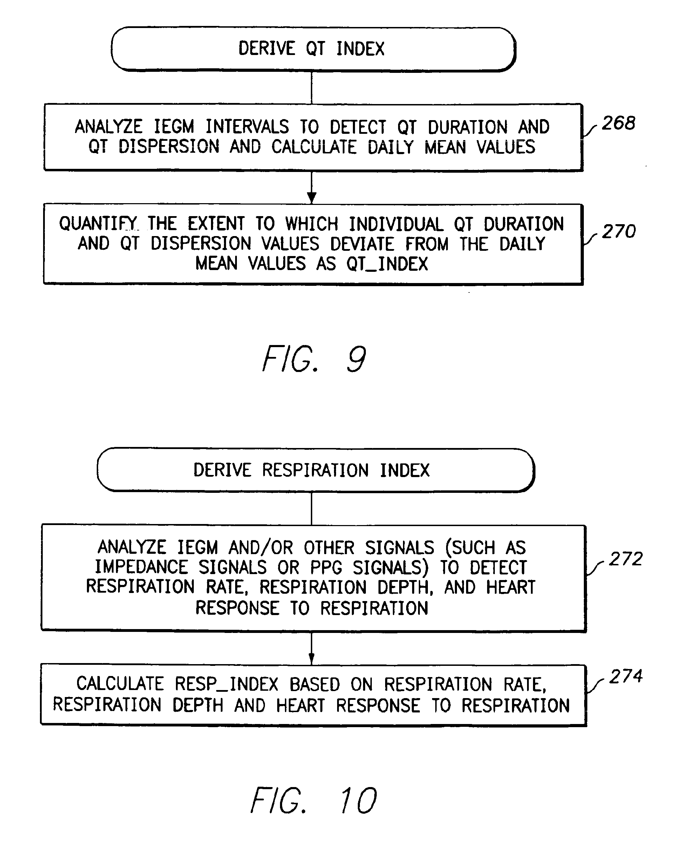

**FIG. 9** summarizes derivation of the QT index. Deep brain infarction can result in a significant dysfunction of the autonomic nervous system, manifesting itself as distorted dynamic behavior of the QT interval and with impaired functional performance. Accordingly, at step 268, the pacer/ICD analyzes the IEGM interval parameters (detected at step 202 of **FIG. 3)** to detect QT duration and QT dispersion and calculate daily mean values of these parameters. Then, at step 270, the pacer/ICD quantifies the extent to which individual QT duration and QT dispersion values deviate from the daily mean values as QT_Index. In one example, the following equation is used to calculate the QT_Index:

$$\text{QT\_Index} =$$

$$QTVI = \log_{10}\left\{ \frac{QT_v/QT_m^2}{HR_v/HR_m^2} \right\}$$

Classically, QT dispersion is defined as the difference between the maximum and minimum QT interval of the 12 lead ECG. For the purposes of the present descriptions, QTdispersion refers to a measure of variance of QT intervals. QTVI can be measured using consecutive 256 beats and a running average can be maintained over 24 hours. It may be calculated as a logarithm of the ratio of normalized QT variance to heart rate variance. QT_Index is, in one example, the difference between the 24 hour-long term QTVI and the instantaneous (i.e. current) QTVI. See, e.g. Berger et al. "Beat-to-Beat QT Interval Variability: Novel Evidence for Repolarization Lability in Ischemic and Nonischemic Dilated Cardiomyopathy", Circulation. 1997;96:1557-1565.

**FIG. 10** summarizes derivation of the respiration index. A unilateral medullary infarction can result in an extremely rapid and shallow tachypnea due to the increase in respiratory drive. The heart rate response to normal breathing, the Valsalva maneuver and tilting are significantly impaired in both hemispheric and brain stem infarctions, due to hypofunction of the parasympatheric nervous system. Accordingly, at step 272, the pacer/ICD analyzes IEGM and/or other signals (such as impedance signals or PPG signals) representative of respiration to detect respiration rate, respiration depth and heart response to respiration. At step 274, the pacer/ICD then calculates RESP_Index as a function of respiration rate, respiration depth and heart response to respiration. In one example, the following equation/procedure is used to calculate the RESP_Index:

$$\text{RESP\_Index} = \sum_{x=1:20} \frac{Te\text{-}i\ (x)}{24hrTe\text{-}i} + \frac{TDe\text{-}i\ (x)}{24hrTDe\text{-}i}$$

where Te-i = Time Interval between inspiration and expiration, which may be calculated over 20 beats and averaged. TDe-i = Depth of respiration calculated as absolute difference between peak expiration and peak inspiration (which may be calculated over 20 beats and averaged.) Resp_Index is expressed as the sum of the above two terms divided by the average interval between inspiration and expiration calculated over a 24 hour interval.

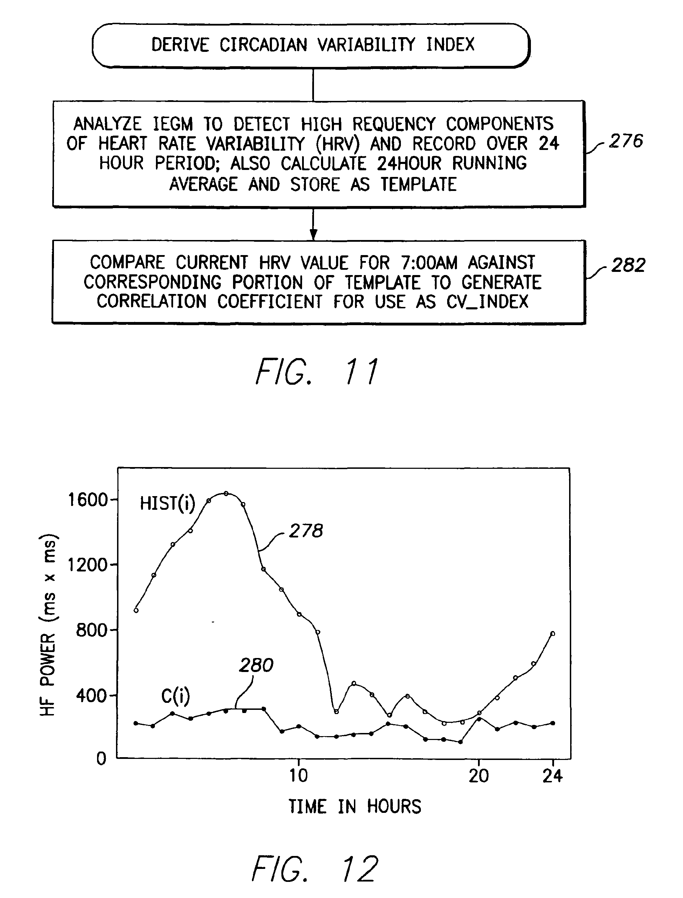

**FIG. 11** summarizes derivation of the circadian variability (CV) index. Circadian fluctuation of heart rate variability is reversibly abolished in the acute phase of ischemic stroke and then it returns during the subsequent six months. The loss of the relative vagal nocturnal dominance may contribute to the incidence of cardiac arrhythmias and other cardiovascular complications after acute stroke. IEGM-based respiration rate and depth variation during a twenty-four hour period can be used to reflect the circadian rhythm variability. Additionally, the HRV high frequency component (discussed above) also reflects the circadian rhythm variability. A running average of twenty-four hour circadian rhythm variability template can be maintained and compared with the current HRV high frequency component, e.g. the HRV feature at 7: 00 am is compared against the corresponding 7:00 am component from the template, and the correlation coefficient forms the CV_Index. As the patient condition improves, the CV index will change and can be used to monitor chronic recovery of the patient.

Accordingly, at step 276, the pacer/ICD analyzes the electrocardiac interval parameters detected at step 202 of **FIG. 3** to extract or derive HRV parameters, which are recorded over a twenty-four hour period. At step 276, the pacer/ICD also calculates a twenty-four hour running average and stores the average as a template.

**FIG. 12** illustrates the circadian variation 278 in the high frequency component of heart rate variability (based on HRV means) in healthy control subjects (Hist(i)). In contrast, curve 280 illustrates the circadian variation in patients with acute brain infarction (C(i)). See, e.g., Korpelainen et al., "Circadian Rhythm of Heart Rate Variability Is Reversibly Abolished in Ischemic Stroke." Stroke. 1997;28:2150-2154. As can be seen, there is relatively little circadian variation in the brain infarction patients. As such, an index representative of the circadian variation can be indicative of stroke.

At step 258, the pacer/ICD compares the current HRV value for a given time (e.g. 7:00 am) against the corresponding portion of the stored template to generate a correlation coefficient for use as CV_Index. In one example, the following equation is used to calculate the CV_Index:

$$\text{CV\_Index} = \text{Absolute (Hist(i) - C(i)) / Integral of Hist(i) over 24 hour period.}$$

As shown in **FIG. 12,** a 24 hour circadian trend can be stored and continuously rebuilt. At any given point in time, the current circadian variable (e.g. HF power) can be compared with the circadian variable 24 hours before. For example, the circadian trend can be derived as a measurement of QTinterval instead of HF power such that the units are in milliseconds. The CV_index can then be calculated as a difference as shown in the equation above, divided by the integral of the circadian trend.

**FIG. 13** summarizes derivation of the composite stroke index based on the various individual indices. At step 284, the pacer/ICD calculates time delays for all individual indices representative of the time rates of change of the indices. In this regard, strokes develop abruptly, hence a measure of the time delay of occurrence of the changes in the various indices is a useful feature. The time delta may be represented as the amount of time it takes for the index to change from its current value to the threshold value. With the device monitoring continuously, the device thereby can record a time trend to determine this information. Hence, for each individual index, e.g. HRV index, if the index changes above a respective threshold (e.g. HRVindexThreshold), the time delta of the change will be recorded by the device as: HRVtime-delta-index. (Particular values for use as the respective thresholds can be determined in advance based on an analysis of the stroke indices for healthy patients as compared with indices for stroke patients.) The time delta index can be quantified as a graded measure from the TIMEdelta on a scale of 0 to 1, as shown in TABLE I:

TABLE I

| Time | Time Delta index |
|---|---|
| 0-10sec | 0.1 |
| 10 sec - 100 sec | .2 |
| 100 sec - 150 sec | .3 |
| 150 sec - 200 sec | .4 |
| 200 sec - 250 sec | .5 |
| 250 sec - 300 sec | .6 |
| 300 sec - 1 day | .7 |
| 1 day - 3 days | .8 |
| 3 days - 1 week | .9 |
| 1 week - 1 month | 1.0 |

At step 286, the pacer/ICD then calculates the composite stroke index as:

Composite Stroke Index =

$$HRV\_Index / HRVtime\_Delta\_Index \quad +$$
$$HRT\_Index / HRTtime\_Delta\_Index \quad +$$
$$BRS\_Index / BRStime\_Delta\_Index \quad +$$
$$QT\_Index / QTtime\_Delta\_Index \quad +$$
$$RESP\_Index / RESPtime\_Delta\_Index \quad +$$
$$CV\_Index / CVtime\_Delta\_Index.$$

As already explained, this composite stroke index is compared against a threshold indicative of stroke to detect a stroke event. (The particular value for use as the composite threshold can be determined in advance based on an analysis of composite stroke indices for healthy patients as compared with indices for stroke patients. The threshold value is then programmed into the pacer/ICD.) The composite index may be used, not only to detect stroke, but to also monitor patient recovery following the stroke. For example, the pacer/ICD can periodically record the stroke index for use a diagnostic tool in evaluating recovery.

As can be appreciated, the particular combination of indices listed at step 286 is only exemplary. Other composite indices can be derived using more or fewer individual indices.

What have been described are various techniques for detecting stoke within a patient. A detailed description of an exemplary pacer/ICD for implementing these techniques will now be provided. However, principles of invention may be implemented within other pacer/ICD implementations or within other implantable devices such as stand-alone stroke monitoring devices, CRT devices or CRT-D devices.

Furthermore, although examples described herein involve processing of data by the implanted device itself to detect stroke, some operations might be performed using an external device, such as a bedside monitor, device programmer, computer server or other external system. For example, recorded data could be transmitted to an external device, which then processes the data to detect stroke. Processing by the implanted device itself is preferred as that allows the device to promptly detect stroke and to issue immediate warnings, deliver therapy, etc.

Exemplary Pacer/ICD

**FIG. 14** provides a simplified block diagram of the pacer/ICD, which is a dual-chamber stimulation device capable of treating both fast and slow arrhythmias with stimulation therapy, including cardioversion, defibrillation, and pacing stimulation, as well as capable of performing the stroke detection functions described above. To provide atrial chamber pacing stimulation and sensing, pacer/ICD 10 is shown in electrical communication with a heart 312 by way of a left atrial lead 320 having an atrial tip electrode 322 and an atrial ring electrode 323 implanted in the atrial appendage. Pacer/ICD 10 is also in electrical communication with the heart by way of a right ventricular lead 330 having, in this embodiment, a ventricular tip electrode 332, a right ventricular ring electrode 334, a right ventricular (RV) coil electrode 336, and a superior vena cava (SVC) coil electrode 338. Typically, the right ventricular lead 330 is transvenously inserted into the heart so as to place the RV coil electrode 336 in the right ventricular apex, and the SVC coil electrode 338 in the superior vena cava. Accordingly, the right ventricular lead is capable of receiving cardiac signals, and delivering stimulation in the form of pacing and shock therapy to the right ventricle.

To sense left atrial and ventricular cardiac signals and to provide left chamber pacing therapy, pacer/ICD 10 is coupled to a "coronary sinus" lead 324 designed for placement in the "coronary sinus region" via the coronary sinus os for positioning a distal electrode adjacent to the left ventricle and/or additional electrode(s) adjacent to the left atrium. As used herein, the phrase "coronary sinus region" refers to the vasculature of the left ventricle, including any portion of the coronary sinus, great cardiac vein, left marginal vein, left posterior ventricular vein, middle cardiac vein, and/or small cardiac vein or any other cardiac vein accessible by the coronary sinus. Accordingly, an exemplary coronary sinus lead 324 is designed to receive atrial and ventricular cardiac signals and to deliver left ventricular pacing therapy using at least a left ventricular tip electrode 326, left atrial pacing therapy using at least a left atrial ring electrode 327, and shocking therapy using at least a left atrial coil electrode 328. With this configuration, biventricular pacing can be performed. Although only three leads are shown in **FIG. 14,** it should also be understood that additional stimulation leads (with one or more pacing, sensing and/or shocking electrodes) may be used in order to efficiently and effectively provide pacing stimulation to the left side of the heart or atrial cardioversion and/or defibrillation.

The leads may be provided with one or more physiological sensors, such as sensor 13 shown attached to lead 330 in the RV.

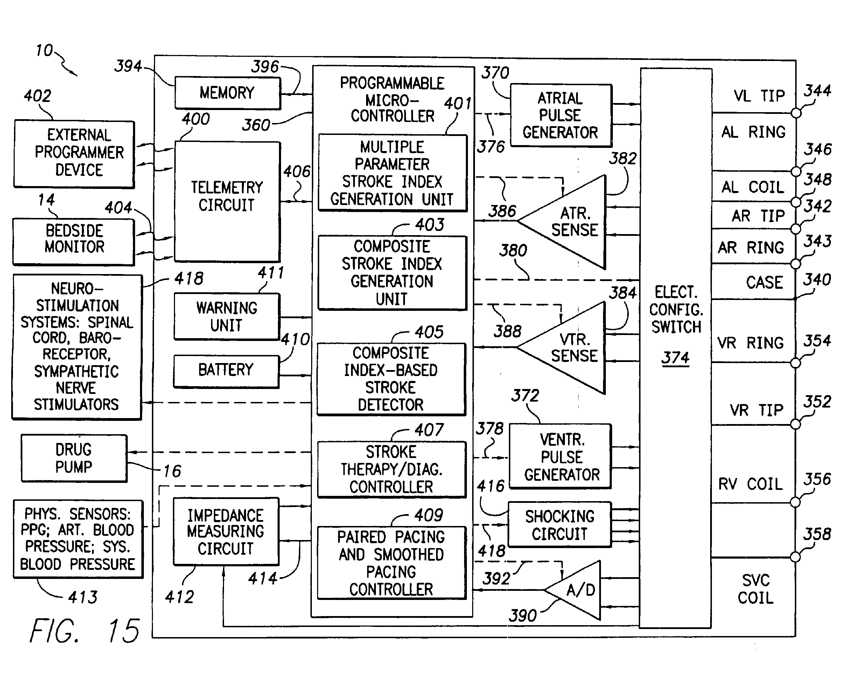

A simplified block diagram of internal components of pacer/ICD 10 is shown in **FIG. 15.** While a particular pacer/ICD is shown, this is for illustration purposes only, and one of skill in the art could readily duplicate, eliminate or disable the appropriate circuitry in any desired combination to provide a device capable of treating the appropriate chamber(s) with cardioversion, defibrillation and pacing stimulation as well as providing for the aforementioned stroke monitoring functions.

The housing 340 for pacer/ICD 10, shown schematically in **FIG. 15,** is often referred to as the "can", "case" or "case electrode" and may be programmably selected to act as the return electrode for all "unipolar" modes. The housing 340 may further be used as a return electrode alone or in combination with one or more of the coil electrodes, 328, 336 and 338, for shocking purposes. The housing 340 further includes a connector (not shown) having a plurality of terminals, 342, 343, 344, 346, 348, 352, 354, 356 and 358 (shown schematically and, for convenience, the names of the electrodes to which they are connected are shown next to the terminals). As such, to achieve right atrial sensing and pacing, the connector includes at least a right atrial tip terminal ($A_R$ TIP) 342 adapted for connection to the atrial tip electrode 322 and a right atrial ring ($A_R$ RING) electrode 343 adapted for connection to right atrial ring electrode 323. To achieve left chamber sensing, pacing and shocking, the connector includes at least a left ventricular tip terminal ($V_L$ TIP) 344, a left atrial ring terminal ($A_L$ RING) 346, and a left atrial shocking terminal ($A_L$ COIL) 348, which are adapted for connection to the left ventricular ring electrode 326, the left atrial tip electrode 327, and the left atrial coil electrode 328, respectively. To support right chamber sensing, pacing and shocking, the connector further includes a right ventricular tip terminal ($V_R$ TIP) 352, a right ventricular ring terminal ($V_R$ RING) 354, a right ventricular shocking terminal ($R_V$ COIL) 356, and an SVC shocking terminal (SVC COIL) 358, which are adapted for connection to the right ventricular tip electrode 332, right ventricular ring electrode 334, the RV coil electrode 336, and the SVC coil electrode 338, respectively.

At the core of pacer/ICD 10 is a programmable microcontroller 360, which controls the various modes of stimulation therapy. As is well known in the art, the microcontroller 360 (also referred to herein as a control unit) typically includes a microprocessor, or equivalent control circuitry, designed specifically for controlling the delivery of stimulation therapy and may further include RAM or ROM memory, logic and timing circuitry, state machine circuitry, and I/O circuitry. Typically, the microcontroller 360 includes the ability to process or monitor input signals (data) as controlled by a program code stored in a designated block of memory. The details of the design and operation of the microcontroller 360 are not critical to the invention. Rather, any suitable microcontroller 360 may be used that carries out the functions described herein. The use of microprocessor-based control circuits for performing timing and data analysis functions are well known in the art.

As shown in **FIG. 15,** an atrial pulse generator 370 and a ventricular/impedance pulse generator 372 generate pacing stimulation pulses for delivery by the right atrial lead 320, the right ventricular lead 330, and/or the coronary sinus lead 324 via an electrode configuration switch 374. It is understood that in order to provide stimulation therapy in each of the four chambers of the heart, the atrial and ventricular pulse generators, 370 and 372, may include dedicated, independent pulse generators, multiplexed pulse generators or shared pulse generators. The pulse generators, 370 and 372, are controlled by the microcontroller 360 via appropriate control signals, 376 and 378, respectively, to trigger or inhibit the stimulation pulses.

The microcontroller 360 further includes timing control circuitry (not separately shown) used to control the timing of such stimulation pulses (e.g., pacing rate, atrio-ventricular (AV) delay, atrial interconduction (A-A) delay, or ventricular interconduction (V-V) delay, etc.) as well as to keep track of the timing of refractory periods, blanking intervals, noise detection windows, evoked response windows, alert intervals, marker channel timing, etc., which is well known in the art. Switch 374 includes a plurality of switches for connecting the desired electrodes to the appropriate I/O circuits, thereby providing complete electrode programmability. Accordingly, the switch 374, in response to a control signal 380 from the microcontroller 360, determines the polarity of the stimulation pulses (e.g., unipolar, bipolar, combipolar, etc.) by selectively closing the appropriate combination of switches (not shown) as is known in the art.

Atrial sensing circuits 382 and ventricular sensing circuits 384 may also be selectively coupled to the right atrial lead 320, coronary sinus lead 324, and the right ventricular lead 330, through the switch 374 for detecting the presence of cardiac activity in each of the four chambers of the heart. Accordingly, the atrial (ATR. SENSE) and ventricular (VTR. SENSE) sensing circuits, 382 and 384, may include dedicated sense amplifiers, multiplexed amplifiers or shared amplifiers. The switch 374 determines the "sensing polarity" of the cardiac signal by selectively closing the appropriate switches, as is also known in the art. In this way, the clinician may program the sensing polarity independent of the stimulation polarity. Each sensing circuit, 382 and 384, preferably employs one or more low power, precision amplifiers with programmable gain and/or automatic gain control, bandpass filtering, and a threshold detection circuit, as known in the art, to selectively sense the cardiac signal of interest. The automatic gain control enables pacer/ICD 10 to deal effectively with the difficult problem of sensing the low amplitude signal characteristics of atrial or ventricular fibrillation. The outputs of the atrial and ventricular sensing circuits, 382 and 384, are connected to the microcontroller 360 which, in turn, are able to trigger or inhibit the atrial and ventricular pulse generators, 370 and 372, respectively, in a demand fashion in response to the absence or presence of cardiac activity in the appropriate chambers of the heart.

For arrhythmia detection, pacer/ICD 10 utilizes the atrial and ventricular sensing circuits, 382 and 384, to sense cardiac signals to determine whether a rhythm is physiologic or pathologic. As used in this section, "sensing" is reserved for the noting of an electrical signal, and "detection" is the processing of these sensed signals and noting the presence of an arrhythmia. The timing intervals between sensed events (e.g., P-waves, R-waves, and depolarization signals associated with fibrillation which are sometimes referred to as "F-waves" or "Fib-waves") are then classified by the microcontroller 360 by comparing them to a predefined rate zone limit (i.e., bradycardia, normal, atrial tachycardia, atrial fibrillation, low rate VT, high rate VT, and fibrillation rate zones) and various other characteristics (e.g., sudden onset, stability, physiologic sensors, and morphology, etc.) in order to determine the type of remedial therapy that is needed (e.g., bradycardia pacing, antitachycardia pacing, cardioversion shocks or defibrillation shocks).

Cardiac signals are also applied to the inputs of an analog-to-digital (A/D) data acquisition system 390. The data acquisition system 390 is configured to acquire intracardiac electrogram signals, convert the raw analog data into a digital signal, and store the digital signals for later processing and/or telemetric transmission to an external device 402. The data acquisition system 390 is coupled to the right atrial lead 320, the coronary sinus lead 324, and the right ventricular lead 330 through the switch 374 to sample cardiac signals across any pair of desired electrodes. The microcontroller 360 is further coupled to a memory 394 by a suitable data/address bus 396, wherein the programmable operating parameters used by the microcontroller 360 are stored and modified, as required, in order to customize the operation of pacer/ICD 10 to suit the needs of a particular patient. Such operating parameters define, for example, the aforementioned thresholds as well as pacing pulse amplitude or magnitude, pulse duration, electrode polarity, rate, sensitivity, automatic features, arrhythmia detection criteria, and the amplitude, waveshape and vector of each shocking pulse to be delivered to the patient's heart within each respective tier of therapy. Other pacing parameters include base rate, rest rate and circadian base rate.

Advantageously, the operating parameters of the implantable pacer/ICD 10 may be non-invasively programmed into the memory 394 through a telemetry circuit 400 in telemetric communication with the external device 402, such as a programmer, transtelephonic transceiver or a diagnostic system analyzer. The telemetry circuit 400 is activated by the microcontroller by a control signal 406. The telemetry circuit 400 advantageously allows intracardiac electrograms and status information relating to the operation of pacer/ICD 10 (as contained in the microcontroller 360 or memory 394) to be sent to the external device 402 through an established communication link 404. Pacer/ICD 10 further includes an accelerometer or other physiologic sensor 408, commonly referred to as a "rate-responsive" sensor because it is typically used to adjust pacing stimulation rate according to the exercise state of the patient. However, the physiological sensor 408 may further be used to detect changes in cardiac output, changes in the physiological condition of the heart, or diurnal changes in activity (e.g., detecting sleep and wake states) and to detect arousal from sleep.

Accordingly, the microcontroller 360 responds by adjusting the various pacing parameters (such as rate, AV Delay, V-V Delay, etc.) at which the atrial and ventricular pulse generators, 370 and 372, generate stimulation pulses. While shown as being included within pacer/ICD 10, it is to be understood that the physiologic sensor 408 may also be external to pacer/ICD 10, yet still be implanted within or carried by the patient. A common type of rate responsive sensor is an activity sensor incorporating an accelerometer or a piezoelectric crystal, which is mounted within the housing 340 of pacer/ICD 10. Other types of physiologic sensors are also known, for example, sensors that sense the oxygen content of blood, respiration rate and/or minute ventilation, pH of blood, ventricular gradient, etc.

The pacer/ICD additionally includes a battery 410, which provides operating power to all of the circuits shown in **FIG. 15.** The battery 410 may vary depending on the capabilities of pacer/ICD 10. If the system only provides low voltage therapy, a lithium iodine or lithium copper fluoride cell may be utilized. For pacer/ICD 10, which employs shocking therapy, the battery 410 must be capable of operating at low current drains for long periods, and then be capable of providing high-current pulses (for capacitor charging) when the patient requires a shock pulse. The battery 410 should also have a predictable discharge characteristic so that elective replacement time can be detected. Accordingly, pacer/ICD 10 is preferably capable of high voltage therapy and appropriate batteries.

As further shown in **FIG. 15,** pacer/ICD 10 is shown as having an impedance measuring circuit 412 which is enabled by the microcontroller 360 via a control signal 414. Uses for an impedance measuring circuit include, but are not limited to, lead impedance surveillance during the acute and chronic phases for proper lead positioning or dislodgement; detecting operable electrodes and automatically switching to an operable pair if dislodgement occurs; measuring respiration or minute ventilation; measuring thoracic impedance for determining shock thresholds; detecting when the device has been implanted; measuring stroke volume; and detecting the opening of heart valves, etc. The impedance measuring circuit 412 is advantageously coupled to the switch 374 so that any desired electrode may be used.

In the case where pacer/ICD 10 is intended to operate as an implantable cardioverter/defibrillator (ICD) device, it detects the occurrence of an arrhythmia, and automatically applies an appropriate electrical shock therapy to the heart aimed at terminating the detected arrhythmia. To this end, the microcontroller 360 further controls a shocking circuit 416 by way of a control signal 418. The shocking circuit 416 generates shocking pulses of low (up to 0.5 joules), moderate

EP 2 294 972 B1

(0.5 - 10 joules) or high energy (11 to 40 joules), as controlled by the microcontroller 360. Such shocking pulses are applied to the heart of the patient through at least two shocking electrodes, and as shown in this embodiment, selected from the left atrial coil electrode 328, the RV coil electrode 336, and/or the SVC coil electrode 338. The housing 340 may act as an active electrode in combination with the RV electrode 336, or as part of a split electrical vector using the SVC coil electrode 338 or the left atrial coil electrode 328 (i.e., using the RV electrode as a common electrode). Cardio-oversion shocks are generally considered to be of low to moderate energy level (so as to minimize pain felt by the patient), and/or synchronized with an R-wave and/or pertaining to the treatment of tachycardia. Defibrillation shocks are generally of moderate to high energy level (i.e., corresponding to thresholds in the range of 11-40 joules), delivered asynchronously (since R-waves may be too disorganized), and pertaining exclusively to the treatment of fibrillation. Accordingly, the microcontroller 360 is capable of controlling the synchronous or asynchronous delivery of the shocking pulses.

Microcontroller 360 also includes various components directed to detecting and responding to stroke. In particular, the microcontroller includes a multiple parameter stroke index generation unit 401 operative to derive a plurality of indices from electrocardiac signals (sensed by the leads) and from physiological signals (sensed by physiologic sensors 413) based on parameters affected by stroke. A composite stroke index generation unit 403 is operative to derive a composite stroke index from the plurality of indices, as discussed above. A composite index-based stroke detector 405 is operative to detect a stroke event within the patient based on the composite stroke index, as already described. Collectively, the composite stroke index generation unit and the composite index-based stroke detector provide an index-based stroke detector operative to derive a plurality of indices from electrocardiac signals and physiological signals based on parameters affected by stroke.

A stroke therapy/diagnostics controller 407 is operative to initiate, adjust or otherwise control therapy in response to stroke, as well as record stroke diagnostics. The stroke therapy controller can control one more neurostimulation systems 418 including spinal cord, baro-receptor, sympathetic nerve stimulators. The stroke therapy controller can also control the aforementioned drug pump 16. Still further, the microcontroller includes a paired pacing and smoothed pacing controller 409 operative to initiate, adjust or otherwise control paired pacing and smoothed pacing in response to stroke. Warning signals may be relayed to the patient via internal warning device 411 or via bedside monitor 14 or programmer 402.

Note that, for simplicity and clarity, some of the components external the pacer/ICD (such as the neuro-stimulators, the drug pump and the physiological sensors) are shown functionally connected to the microcontroller via phantom lines. In practice, additional electrical connection terminals (not shown) may be needed to connect these components to the pacer/ICD.

Note also that, depending upon the implementation, the various components of the microcontroller may be implemented as separate software modules or the modules may be combined to permit a single module to perform multiple functions. In addition, although shown as being components of the microcontroller, some or all of these components may be implemented separately from the microcontroller.

The invention may be realised using other implantable systems or in accordance with other techniques. Thus, while the invention has been described with reference to particular exemplary embodiments, modifications can be made thereto without departing from the scope of the invention as defined in the appended claims. Note also that the term "including" as used herein is intended to be inclusive, i.e. "including but not limited to."

## Claims

1. A system (8) for detecting a stroke event, the system comprising:

    an implantable medical device (10) for implantation within a patient;
    means (12) configured for sensing electrocardiac signals within the patient;
    means (13) configured for sensing physiological signals within the patient;
    means configured for deriving a plurality of indices from the electrocardiac signals and the physiological signals based on parameters affected by stroke; and
    means configured for detecting a stroke event within the patient based on an examination of the plurality of indices,
    wherein the plurality of indices are selected from a heart rate variability index (HRV), a heart rate turbulence index (HRT), a baroreflex sensitivity index (BRS), a QT index and a respiration index.

2. A system according to claim 1, wherein:

    the means for deriving a plurality of indices comprises a multiple parameter stroke index generation unit (401)

operative to derive a plurality of indices from electrocardiac signals and physiological signals based on parameters affected by stroke; and

the means for detecting a stroke event comprises an index-based stroke detector (405) operative to detect a stroke event within the patient based on an examination of the plurality of indices.

3. A system according to claim 1 or claim 2, the system further comprising:

control means arranged to control at least one device function in response the indication of the stroke.

4. The system of claim1, wherein the heart rate variability (HRV) index is derived from R-R intervals (RRI) detected within the electrocardiac signals.

5. The system of claim1, wherein the physiological signals include an arterial blood pressure signal and wherein the heart rate turbulence (HRT) index is derived, in part, from the arterial pressure signal.

6. The system of claim1, wherein the physiological signals include a systolic blood pressure signal and wherein the baroreflex_(BRS) index is derived from the systolic blood pressure signal and from R-R intervals detected within the electrocardiac signals.

7. The system of claim1, wherein the QT index is derived from intervals between QRS-complexes and T-waves detected within the electrocardiac signals.

8. The system of claim1, wherein respiration parameters are derived and wherein the respiration index is derived from the respiration parameters in combination with heart rate response to respiration derived from the electrocardiac signals.

9. The system of any preceding claim, wherein the plurality of indices additionally include a circadian variability (CV) index or time delta indices representative of time rates of change of the other indices.

10. The system of any preceding claim, wherein detecting a stroke event based on an examination of the plurality of indices includes:

deriving a composite stroke index from the plurality of indices; and
detecting the indication of stroke based on changes in the composite stroke index.

11. The system of claim 10, wherein deriving a composite stroke index includes calculating:

Composite Stroke Index =

$$\text{HRV\_Index} / \text{HRVtime\_Delta\_Index} \quad +$$
$$\text{HRT\_Index} / \text{HRTtime\_Delta\_Index} \quad +$$
$$\text{BRS\_Index} / \text{BRStime\_Delta\_Index} \quad +$$
$$\text{QT\_Index} / \text{QTtime\_Delta\_Index} \quad +$$
$$\text{RESP\_Index} / \text{RESPtime\_Delta\_Index} \quad +$$
$$\text{CV\_Index} / \text{CVtime\_Delta\_Index},$$

wherein HRV_Index is a heart rate variability index, HRVtime_Delta_Index is a time delta for the heart rate variability index, HRT_Index is a heart rate turbulence index, HRTtime_Delta_Index is a time delta for the heart rate turbulence index, BRS_Index is a baroreflex index, BRStime_Delta_Index is a time delta for the baroreflex index, QT_Index is a QT index, QTtime_Delta_Index is a time delta for the QT index, RESP_Index is a respiration index, RESPtime_Delta_Index is a time delta for the respiration index, CV_Index is a circadian variability index, and CVtime_Detta_Index is a time delta for the circadian variability index.

**EP 2 294 972 B1**

12. The system of claim 10, wherein detecting the indication of stroke based on changes in the composite stroke index includes comparing the composite stroke index against a threshold indicative of the onset of a stroke.

13. The system of claim 3, wherein controlling at least one device function in response the indication of the stroke includes generating a warning signal indicative of stroke or selectively delivering neurostimulation to the patient.

14. The system of claim 3, wherein controlling at least one device function in response the indication of the stroke includes selectively delivering neurostimulation to the patient, preferably wherein neurostimulation is delivered via one or more of spinal cord stimulation, baro-receptor stimulation, sympathetic nerve stimulation and a sympathetic blockade stimulation.

15. The system of claim 3, wherein controlling at least one device function in response the indication of the stroke includes controlling pacing in an effort to improve heart rate, preferably controlling pacing includes activating one or more of rate smoothing and paired pacing or controlling pacing is performed in conjunction with real-time changes in a physiological signal detected within the patient.

16. The system of claim 3, wherein the device is equipped with an implantable medication delivery system and wherein controlling at least one device function in response the indication of the stroke includes delivering one or more medications.

**Patentansprüche**

1. System (8) zum Feststellen eines Schlaganfallereignisses, welches System aufweist:

   eine implantierbare medizinische Einrichtung (10) zur Implantation in einen Patienten;
   Mittel (12), die konfiguriert sind, bei dem Patienten elektrokardiale Signale abzufühlen;
   Mittel (13), die konfiguriert sind, bei dem Patienten physiologische Signale abzufühlen;
   Mittel, die konfiguriert sind, aus den elektrokardialen Signalen und den physiologischen Signalen auf Basis von durch den Schlaganfall beeinflussten Parametern eine Mehrzahl von Indizes abzuleiten; und
   Mittel, die konfiguriert sind, auf Basis einer Prüfung der Mehrzahl von Indizes ein Schlaganfallereignis bei dem Patienten festzustellen,
   wobei die Mehrzahl von Indizes ausgewählt ist aus dem Herzfrequenzvariabilitätsindex (HRV), dem Herzfrequenzturbulenzindex (HRT), dem Baroreflex-Sensitivitätsindex (BRS), dem QT-Index und dem Respirationsindex.

2. System nach Anspruch 1, wobei
   die Mittel zum Ableiten einer Mehrzahl von Indizes eine Einheit (401) zur Erzeugung eines Mehrfachparameter-Schlaganfallindexes aufweisen, die wirksam ist, um aus elektrokardialen Signalen und physiologischen Signalen auf Basis von durch den Schlaganfall beeinflussten Parametern eine Mehrzahl von Indizes abzuleiten; und
   die Mittel zum Feststellen eines Schlaganfallereignisses einen indexbasierten Schlaganfalldetektor (405) aufweisen, der wirksam ist, um auf Basis einer Prüfung der Mehrzahl von Indizes ein Schlaganfallereignis bei dem Patienten festzustellen.

3. System nach Anspruch 1 oder 2, welches System weiters aufweist:

   Regelungsmittel, die eingerichtet sind, mindestens eine Vorrichtungsfunktion in Reaktion auf Anzeichen eines Schlaganfalls zu regeln.

4. System nach Anspruch 1, wobei der Herzfrequenzvariabilitätsindex (HRV) aus R-R-Intervallen (RRI) abgeleitet wird, die in den elektrokardialen Signalen detektiert werden.

5. System nach Anspruch 1, wobei die physiologischen Signale ein arterielles Blutdrucksignal enthalten, und wobei der Herzfrequenzturbulenzindex (HRT) teilweise aus dem arteriellen Blutdrucksignal abgeleitet wird.

6. System nach Anspruch 1, wobei die physiologischen Signale ein systolisches Blutdrucksignal enthalten, und wobei der Baroreflex-(BRS)-Index aus dem systolischen Blutdrucksignal und aus R-R-Intervallen abgeleitet wird, die in den elektrokardialen Signalen detektiert werden.

**15**

7. System nach Anspruch 1, wobei der QT-Index aus Intervallen zwischen QRS-Komplexen und T-Wellen abgeleitet wird, die in den elektrokardialen Signalen detektiert werden.

8. System nach Anspruch 1, wobei Respirationsparameter abgeleitet werden, und wobei der Respirationsindex aus den Respirationsparametern in Kombination mit der Herzfrequenzreaktion auf die von den elektrokardialen Signalen abgeleitete Respiration abgeleitet wird.

9. System nach einem der vorhergehenden Ansprüche, wobei die Mehrzahl von Indizes zusätzlich den tagesperiodischen Variabilitätsindex (CV) oder Zeit-Delta-Indizes enthalten, die für Zeitänderungsraten der anderen Indizes stehen.

10. System nach einem der vorhergehenden Ansprüche, wobei das Feststellen eines Schlaganfallereignisses auf Basis einer Prüfung der Mehrzahl von Indizes folgendes umfasst:

Ableiten eines zusammengesetzten Schlaganfallindexes aus der Mehrzahl von Indizes; und
Feststellen des Hinweises auf einen Schlaganfall auf Basis von Veränderungen des zusammengesetzten Schlaganfallindexes.

11. System nach Anspruch 10, wobei das Ableiten eines zusammengesetzten Schlaganfallindexes das Berechnen des

```
zusammengesetzten Schlaganfallindexes =
    HRV_Index  / HRV-Zeit_Delta_Index      +
    HRT_Index  / HRT-Zeit_Delta_Index      +
    BRS_Index  / BRS-Zeit_Delta_Index      +
    QT_Index   / QT-Zeit_Delta_Index       +
    RESP_Index / RESP-Zeit_Delta_Index     +
    CV_Index   / CV-Zeit_Delta_Index       +
```

umfasst, worin der HRV_Index ein Herzfrequenzvariabilitätsindex, der HRV-Zeit_Delta_Index ein Zeitdelta für den Herzfrequenzvariabilitätsindex, der HRT_Index ein Herzfrequenzturbulenzindex, der HRT-Zeit_Delta_Index ein Zeitdelta für den Herzfrequenzturbulenzindex, der BRS_Index ein Baroreflexindex, der BRS-Zeit_Delta_Index ein Zeitdelta für den Baroreflexindex, der QT_Index ein QT-Index, der QT-Zeit_Delta_Index ein Zeitdelta für den QT-Index, der RESP_Index ein Respirationsindex, der RESP-Zeit_Deltaindex ein Zeitdelta für den Respirationsindex, der CV_Index ein tagesperiodischer Variabilitätsindex und der CV-Zeit_Delta_Index ein Zeitdelta für den tagesperiodischen Variabilitätsindex ist.

12. System nach Anspruch 10, wobei das Feststellen des Hinweises auf einen Schlaganfall auf Basis von Veränderungen des zusammengesetzten Schlaganfallindexes das Vergleichen des zusammengesetzten Schlaganfallindexes mit einem Schwellenwert umfasst, der auf einen beginnenden Schlaganfall hinweist.

13. System nach Anspruch 3, wobei das Regeln mindestens einer Vorrichtungsfunktion in Reaktion auf Anzeichen eines Schlaganfalls das Generieren eines Warnsignals, welches auf einen Schlaganfall hinweist, oder das selektive Senden einer Neurostimulation an den Patienten umfasst.

14. System nach Anspruch 3, wobei das Regeln mindestens einer Vorrichtungsfunktion in Reaktion auf Anzeichen eines Schlaganfalls das selektive Senden einer Neurostimulation an den Patienten umfasst, wobei die Neurostimulation vorzugsweise über eine Rückenmarkstimulation, eine Barorezeptorstimulation, eine sympathische Nervenstimulation und/oder eine sympathische Blockadestimulation gesendet wird.

15. System nach Anspruch 3, wobei das Regeln mindestens einer Vorrichtungsfunktion in Reaktion auf Anzeichen eines Schlaganfalls das Regeln der Schrittsteuerung in einem Versuch, die Herzfrequenz zu verbessern, umfasst, wobei das Regeln der Schrittsteuerung vorzugsweise das Aktivieren der Frequenzberuhigung und/oder der paarweisen Schrittsteuerung umfasst oder das Regeln der Schrittsteuerung in Verbindung mit Echtzeitänderungen eines physiologischen Signals durchgeführt wird, das bei einem Patienten detektiert wird.

**16.** System nach Anspruch 3, wobei die Vorrichtung mit einem implantierbaren Medikamentenverabreichungssystem ausgestattet ist und das Regeln mindestens einer Vorrichtungsfunktion in Reaktion auf Anzeichen eines Schlaganfalls das Verabreichen eines oder mehrerer Medikamente umfasst.

**Revendications**

**1.** Système (8) pour détecter un accident vasculaire cérébral, le système comprenant :

un dispositif médical implantable (10) destiné à être implanté à l'intérieur d'un patient ;
des moyens (12) configurés pour détecter des signaux électrocardiographiques à l'intérieur du patient ;
des moyens (13) configurés pour détecter des signaux physiologiques à l'intérieur du patient ;
des moyens configurés pour obtenir une pluralité d'indices à partir des signaux électrocardiographiques et des signaux physiologiques sur la base des paramètres affectés par l'accident vasculaire cérébral ; et
des moyens configurés pour détecter un accident vasculaire cérébral à l'intérieur du patient sur la base d'un examen de la pluralité d'indices,
dans lequel la pluralité d'indices sont choisis parmi un indice de variabilité de la fréquence cardiaque (VFC), un indice de turbulence de la fréquence cardiaque (HRT), un indice de sensibilité du baroréflexe (SBR), un indice QT et un indice respiratoire.

**2.** Système selon la revendication 1, dans lequel :

les moyens pour obtenir une pluralité d'indices comprennent une unité de génération d'index systolique à paramètres multiples (401) utilisée pour obtenir une pluralité d'indices à partir de signaux électrocardiographiques et des signaux physiologiques sur la base des paramètres affectés par l'accident vasculaire cérébral ; et
les moyens pour détecter un accident vasculaire cérébral comprennent un détecteur d'accident vasculaire cérébral à base d'indices (405) utilisé pour détecter un accident vasculaire cérébral à l'intérieur du patient sur la base d'un examen de la pluralité d'indices.

**3.** Système selon la revendication 1 ou la revendication 2, le système comprenant en outre :

des moyens de commande conçus pour commander au moins une fonction de dispositif en réponse à l'indication de l'accident vasculaire cérébral.

**4.** Système selon la revendication 1, dans lequel l'indice de variabilité de la fréquence cardiaque (VFC) est obtenu à partir des intervalles R-R (IRR) détectés à l'intérieur des signaux électrocardiographiques.

**5.** Système selon la revendication 1, dans lequel les signaux physiologiques comprennent un signal de tension artérielle et dans lequel l'indice de turbulence de la fréquence cardiaque (HRT) est obtenu, en partie, à partir du signal de tension artérielle.

**6.** Système selon la revendication 1, dans lequel les signaux physiologiques comprennent un signal de tension artérielle systolique et dans lequel l'indice de baroréflexe (SBR) est obtenu à partir du signal de tension artérielle systolique et à partir des intervalles R-R détectés à l'intérieur des signaux électrocardiographiques.

**7.** Système selon la revendication 1, dans lequel l'indice QT est obtenu à partir des intervalles entre les complexes QRS et les ondes T détectés à l'intérieur des signaux électrocardiographiques.

**8.** Système selon la revendication 1, dans lequel les paramètres respiratoires sont obtenus et dans lequel l'indice respiratoire est obtenu à partir des paramètres respiratoires en combinaison avec la réponse de fréquence cardiaque à la respiration obtenus à partir des signaux électrocardiographiques.

**9.** Système selon l'une quelconque des revendications précédentes, dans lequel la pluralité d'indices comprennent en outre un indice de variabilité du rythme circadien (CV) ou des indices delta temporels représentatifs des fréquences temporelles de changement des autres indices.

**10.** Système selon l'une quelconque des revendications précédentes, dans lequel la détection d'un accident vasculaire cérébral sur la base d'un examen de la pluralité d'indices consiste à :

obtenir un indice systolique composite à partir de la pluralité d'indices ; et
détecter l'indication de l'accident vasculaire cérébral sur la base de changements de l'indice systolique composite.

11. Système selon la revendication 10, dans lequel l'obtention d'un indice systolique composite consiste à calculer :

```
Indice systolique composite =
HRV_Index/HRVtime_Delta-Index +
HRT_Index/HRTtime_Delta-Index +


BRS_Index/BRStime_Delta-Index +
QT_Index/QTtime_Delta-Index +
RESP_Index/RESPtime_Delta-Index +
CV_Index/CVtime_Delta-Index,
```

dans lequel HRV_Index est un indice de variabilité de la fréquence cardiaque, HRVtime_Delta_Index est un delta temporel pour l'indice de variabilité de la fréquence cardiaque, HRT_Index est un indice de turbulence de la fréquence cardiaque, HRTtime_Delta_Index est un delta temporel pour l'indice de turbulence de la fréquence cardiaque, BRS_Index est un indice de baroréflexe, BRStime_Delta_Index est un delta temporel pour l'indice de baroréflexe, QT_Index est un indice QT, QTtime_Delta_Index est un delta temporel pour l'indice QT, RESP_Index est un indice respiratoire, RESPtime_Delta_Index est un delta temporel pour l'indice respiratoire, CV_Index est un indice de variabilité du rythme circadien et CVtime_Delta_Index est un delta temporel pour l'indice de variabilité du rythme circadien.

12. Système selon la revendication 10, dans lequel la détection de l'indication d'accident vasculaire cérébral basée sur les changements de l'indice systolique composite consiste à comparer l'indice systolique composite à un seuil indicatif du début d'un accident vasculaire cérébral.

13. Système selon la revendication 3, dans lequel la commande d'au moins une fonction de dispositif en réponse à l'indication de l'accident vasculaire cérébral consiste à générer un signal d'avertissement indicatif de l'accident vasculaire cérébral ou à délivrer sélectivement une neurostimulation au patient.

14. Système selon la revendication 3, dans lequel la commande d'au moins une fonction de dispositif en réponse à l'indication de l'accident vasculaire cérébral consiste à délivrer sélectivement une neurostimulation au patient, la neurostimulation étant délivrée de préférence par le biais d'une ou plusieurs parmi une stimulation de la moelle épinière, une stimulation des barorécepteurs, une stimulation du nerf sympathique et une stimulation du blocage sympathique.

15. Système selon la revendication 3, dans lequel la commande d'au moins une fonction de dispositif en réponse à l'indication de l'accident vasculaire cérébral consiste à commander la stimulation dans un effort pour améliorer la fréquence cardiaque, la commande de la stimulation consistant de préférence à activer un ou plusieurs du lissage de la fréquence et de la stimulation appariée ou la commande de la stimulation étant réalisée conjointement avec les changements en temps réel d'un signal physiologique détecté à l'intérieur du patient.

16. Système selon la revendication 3, dans lequel le dispositif est équipé d'un système d'administration de médicament implantable et dans lequel la commande d'au moins une fonction de dispositif en réponse à l'indication de l'accident vasculaire cérébral consiste à administrer un ou plusieurs médicaments.

*8*

18

NEURO-
STIMULATOR

PHYS.
SENSOR    *12*

*13*

RV
LEAD

*10*

LV
LEAD    DRUG
PUMP

*16*

PACER/ICD WITH
WITH INDEX-
BASED STROKE
DETECTION
SYSTEM

BEDSIDE MONITOR
OR OTHER
EXTERNAL DEVICE

*14*

*FIG. 1*

OVERVIEW OF INDEX-BASED
CEREBRAL STROKE CONFIRMATION
USING AN IMPLANTABLE MEDICAL
DEVICE

SENSE ELECTROCARDIAC AND
PHYSIOLOGICAL SIGNALS WITHIN
THE PATIENT — 100

DERIVE A PLURALITY OF INDICES
FROM THE ELECTROCARDIAC SIGNALS
AND THE PHYSIOLOGICAL SIGNALS
BASED ON SIGNAL PARAMETERS
AFFECTED BY STROKE — 102

DETECT A STROKE EVENT WITHIN THE
PATIENT BASED ON AN EXAMINATION
OF THE PLURALITY OF INDICES — 104

GENERATE WARNING, DELIVER
NEUROSTIMULATION, ADJUST PACING,
DELIVER MEDICATIONS AND/OR
CONTROL OTHER DEVICE FUNCTIONS
IN RESPONSE TO THE STROKE — 106

FIG. 2

**FIG. 3**

INDEX-BASED STROKE DETECTION EXAMPLE

RECORD AND STORE IEGMS FROM RIGHT ATRIUM, LEFT ATRIUM, RIGHT VENTRICLE AND LEFT VENTRICLE — 200

DERIVE ELECTROCARDIAC INTERVAL PARAMETERS FROM ALL CHAMBERS; RR INTERVAL, QT INTERVAL, QRS WIDTH, T-WAVE WIDTH, ETC. — 202

DERIVE MORPHOLOGICAL PARAMETERS FROM ALL RECORDED IEGM: QRS MORPHOLOGY, P-WAVE MORPHOLOGY, T-WAVE MORPHOLOGY, ETC. — 204

DETECT AND RECORD PHYSIOLOGICAL SENSOR PARAMETERS SUCH AS ARTERIAL AND SYSTOLIC BLOOD PRESSURE, RESPIRATION RATE, AND RESPIRATION DEPTH — 206

FROM THE VARIOUS PARAMETERS, DERIVE:

HEART RATE VARIABILITY INDEX (HRV_INDEX) (FIG.4);
HEART RATE TURBULENCE INDEX (HRT_INDEX) (FIG. 7);
BAROREFLEX INDEX (BRS_INDEX) (FIG.8);
QT INDEX (QT_INDEX) (FIG.9);
RESPIRATION INDEX (RESP_INDEX) (FIG.10);
CIRCADIAN VARIABILITY INDEX (CV_INDEX) FIG.11); AND
TIME DELTA INDICES FOR EACH RESPECTIVE INDEX — 208

GENERATE A COMPOSITE STROKE INDEX BASED ON THE INDIVIDUAL INDICES AND THEIR RESPECTIVE TIME DELTAS — 210

COMPARE COMPOSITE INDEX AGAINST A THRESHOLD INDICATIVE OF A STROKE EVENT AND GENERATE WARNINGS, DELIVER NEUROSTIMULATION (VIA SPINAL CORD, BARO-RECEPTORS OR SYMPATHETIC NERVES), DELIVER SMOOTHED OR PARIED PACING, DELIVER MEDICATIONS, ETC., IN RESPONSE TO THE STROKE AND IN CONJUNCTION WITH REAL-TIME BLOOD PRESSURE CHANGES, RECORD DIAGNOSTICS, AND MONITOR RECOVERY — 212

DERIVE HEART RATE
VARIABILITY INDEX

ANALYZE THE ELECTROCARDIAC INTERVAL PARAMETERS
TO DERIVE:

RR INTERVALS (RRI);
STANDARD—DEVIATION OF THE RR INTERVALS (SDNN);
VERY LOW FREQUENCY POWER (VLF_POWER);
LOW FREQUENCY POWER (LF_POWER); AND
HIGH FREQUENCY POWER (HF_POWER)

/ 252

CALCULATE HRV_INDEX FROM RRI, SDNN, VLF_POWER,
LF_POWER AND HF_POWER

/ 258

FIG. 4

FIG. 5

FIG. 6

DERIVE HEART RATE
TURBULENCE INDEX

ANALYZE IEGM TO DETECT PREMATURE VENTRICULAR
CONTRACTIONS (PVCs) ⌐260

EXAMINE ARTERIAL PRESSURE TO DETECT HRT RESPONSE
TO PVC THEN QUANTIFY THE RESPONSE AS THE HRT_INDEX ⌐262

*FIG. 7*

DERIVE BAROREFLEX INDEX

ANALYZE IEGM INTERVAL PARAMETERS TO DETECT
CHANGES IN RR INTERVALS AND ANALYZE PHYSIOLOGICAL
SIGNALS TO DETECT CHANGES IN SYSTOLIC BLOOD
PRESSURE WITHIN A MID-FREQUENCY BAND (0.07-0.14 HZ) ⌐264

CALCULATE BRS_INDEX AS RATIO OF CHANGES IN RR
INTERVALS TO CHANGES IN SYSTOLIC BLOOD PRESSURE ⌐266

*FIG. 8*

DERIVE QT INDEX

ANALYZE IEGM INTERVALS TO DETECT QT DURATION AND QT DISPERSION AND CALCULATE DAILY MEAN VALUES — 268

QUANTIFY THE EXTENT TO WHICH INDIVIDUAL QT DURATION AND QT DISPERSION VALUES DEVIATE FROM THE DAILY MEAN VALUES AS QT_INDEX — 270

FIG. 9

DERIVE RESPIRATION INDEX

ANALYZE IEGM AND/OR OTHER SIGNALS (SUCH AS IMPEDANCE SIGNALS OR PPG SIGNALS) TO DETECT RESPIRATION RATE, RESPIRATION DEPTH, AND HEART RESPONSE TO RESPIRATION — 272

CALCULATE RESP_INDEX BASED ON RESPIRATION RATE, RESPIRATION DEPTH AND HEART RESPONSE TO RESPIRATION — 274

FIG. 10

DERIVE CIRCADIAN VARIABILITY INDEX

ANALYZE IEGM TO DETECT HIGH REQUENCY COMPONENTS OF HEART RATE VARIABILITY (HRV) AND RECORD OVER 24 HOUR PERIOD; ALSO CALCULATE 24HOUR RUNNING AVERAGE AND STORE AS TEMPLATE — 276

COMPARE CURRENT HRV VALUE FOR 7:00AM AGAINST CORRESPONDING PORTION OF TEMPLATE TO GENERATE CORRELATION COEFFICIENT FOR USE AS CV_INDEX — 282

*FIG. 11*

*FIG. 12*

GENERATE COMPOSITE STROKE
INDEX

CALCULATE TIME DELAYS FOR ALL INDIVIDUAL INDICES
REPRESENTATIVE OF THE TIME RATES OF CHANGE OF THE
INDICES /284

CALCULATE COMPOSITE STROKE INDEX AS: /286

HRV_INDEX / HRVTIME_DELTA_INDEX     +
HRT_INDEX / HRTTIME_DELTA_INDEX     +
BRS_INDEX / QTTIME_DELTA_INDEX     +
RESP_INDEX / RESPTIME_DELTA_INDEX     +
CV_INDEX / CV TIME_DELTA_INDEX

FIG. 13

FIG. 14

FIG. 15

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 102007045738 A
- EP 2215965 A
- US 5328460 A, Lord
- US 92702607 A
- US 37860406 A, Kroll
- US 62366307 A, Zou
- US 7361146 B, Bharmi
- US 6449509 B, Park

- US 6064910 A, Andersson
- US 7099718 B, Thacker
- US 6050952 A, Hakki
- US 6937896 B, Kroll
- US 6636766 B, Bornzin
- US 2010094371 A, Bornzin
- US 7235530 B, Blair


**Non-patent literature cited in the description**

- **KORPELAINEN et al.** Abnormal Heart Rate Variability as a Manifestation of Autonomic Dysfunction in Hemispheric Brain Infarction. *Stroke,* 1996, vol. 27, 2059-2063
- **WATANABE et al.** Effects of ventricular premature stimulus coupling interval on blood pressure and heart rate turbulence. *Circulation,* 16 July 2002, vol. 106 (3), 325-30

- **BERGER et al.** Beat-to-Beat QT Interval Variability: Novel Evidence for Repolarization Lability in Ischemic and Nonischemic Dilated Cardiomyopathy. *Circulation,* 1997, vol. 96, 1557-1565
- **KORPELAINEN et al.** Circadian Rhythm of Heart Rate Variability Is Reversibly Abolished in Ischemic Stroke. *Stroke,* 1997, vol. 28, 2150-2154